Europäisches Patentamt

European Patent Office

Office européen des brevets

(19)

(11) Publication number: **0 223 932 B1**

# (12) EUROPEAN PATENT SPECIFICATION

(45) Date of publication of patent specification: **10.07.91**

(51) Int. Cl.⁵: **G01N 31/00, G01N 33/00**

(21) Application number: **86111874.3**

(22) Date of filing: **27.08.86**

(54) **Sensitivity-calibration circuit for HC analyzers.**

(30) Priority: **27.11.85 JP 183440/85 U**

(43) Date of publication of application:
**03.06.87 Bulletin 87/23**

(45) Publication of the grant of the patent:
**10.07.91 Bulletin 91/28**

(84) Designated Contracting States:
**AT BE CH DE FR GB IT LI NL SE**

(56) References cited:
EP-A- 0 067 931     DE-A- 1 673 182
DE-A- 2 636 500     US-A- 4 151 738
US-A- 4 167 665     US-A- 4 494 399

(73) Proprietor: **HORIBA, LTD.**
**2 Miyanohigashi-machi Kissyoin**
**Minami-ku Kyoto(JP)**

(72) Inventor: **Kohsaka, Hiroji**
**1-56, 2 chome Nishi-shibukawa**
**Kusatsu-city Shiga-prefecture(JP)**
Inventor: **Kihara, Nobutaka**
**4-24, 1 chome Chiyogaoka**
**Nara(JP)**
Inventor: **Nakajima, Yoshiyuki**
**37, Daitokuji-cho Murasakino**
**Kita-ku Kyoto(JP)**
Inventor: **Kojima, Kennosuke**
**46-9, Fujimidai**
**Ohtsu-city Shiga-prefecture(JP)**

(74) Representative: **TER MEER - MÜLLER - STEIN-**
**MEISTER & PARTNER**
**Mauerkircherstrasse 45**
**W-8000 München 80(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 223 932 B1

## Description

The invention relates to a sensitivity-calibration circuit as claimed in the preamble of claim 1 used in HC analyzers for detecting the presence and concentration of "HC gases" such as methane ($CH_4$) and ethane ($C_2H_6$).

According to "Analytical Procedure for Continuous Measurement of Carbon Monoxide, Carbon Dioxide and Hydrocarbon in Automobile Exhaust Gas", Japan. Ind. Standard, Tokyo, Japan, 1976, D1030, 1976, p.17, the sensitivity-calibration of HC analyzers has been carried out as follows:

At first, during the initial adjustment of a HC analyzer, immediately after an appointed sensitivity-calibration has been carried out by using a standard-n-hexane having known concentration standard, propane gas having also a known concentration $C_p$ is flown through the HC analyzer to measure the concentration thereof. A scale factor $P_c$ inherent to said analyzer for determining a n-hexane gas-concentration reduced value $C_N$ of propane gas is determined from an output of the HC analyzer, and the scale factor $P_c$ (for example "the scale factor for a n-hexane gas-concentration" : 0.521) is recorded on a display label (or the like) and this display label is stuck to the HC analyzer.

However, n-hexane gas, which is used as a standard for the calibration, has inherently and always the unstable property of being remarkably apt to be liquefied. Therefore, this standard n-hexane has been substituted by a standard propane gas having a known concentration for the usual calibration after the above described initial adjustment.

During the usual calibration following an initial adjustment, at first the n-hexane gas-concentration reduced value $C_N$ of the standard propane gas is determined according to the following equation on the basis of the known concentration $C_p$ of the standard propane gas. This concentration value is taken from a display label (or the like) which is fixed to a gas cylinder showing for example the user information: "concentration: 1,862 ppm $C_3H_8$". In addition, the scale factor $P_c$ inherent to the HC analyzer is needed which has been previously recorded and can be taken by the user from the display label (or the like) affixed to the HC analyzer as mentioned above.

n-hexane gas-concentration reduced value $C_N$
= (concentration $C_p$ of standard propane gas) x (scale factor $P_c$)
(= 1.862 x 0.521 = about 0.970)

And then, the standard propane gas is flown through the HC analyzer and the sensitivity-calibration circuit in the HC analyzer is adjusted so that an output of the HC analyzer may indicate the n-hexane gas-concentration reduced value $C_N$ (for example 970 ppm) determined in the above described manner.

However, the above described conventional means, since each HC analyzer has a scale factor $P_c$ which is inherent and different due to differences in the optical system, electric circuits and the like, has the disadvantage that a calculation of multiple figures on the basis of the above described equation must be carried out in every usual calibration after the initial adjustment. This is remarkably troublesome and time consuming and requires experienced personel.

On the background of the above described actual state of the art, it is an object of the present invention to provide a sensitivity-calibration circuit for HC analyzers enabling a simple operation of setting the scale factor $P_c$ in the HC analyzer at a specified numerical value which can be easily calculated, so that the calculation of determining a n-hexane gas-concentration reduced value $C_N$ of a standard propane used for the calibration may be carried out much quicker.

In order to achieve the above described object, a sensitivity-calibration circuit for a hydrocarbon (HC) analyzer used for detecting the concentration of HC gases as an n-hexane reduced gas concentration $C_N$ of propane gas, which circuit is connected to an output of said analyzer and comprising a sensitivity adjusting means for adjusting the sensitivity of said analyzer, is characterized in that said circuit further comprises a scale factor adjusting means for setting a scale factor $P_C$ of said analyzer and for determining said n-hexane reduced gas concentration value $C_N$ of propane gas, said scale factor adjusting means being coupled to said sensitivity adjusting means and to a switching means for switching said circuit between a first branch including said scale factor adjusting means and a second branch.

The advantageous effects resulting from the above characteristical construction are as follows:

With the sensitivity-calibration circuit according to the present invention, since scale factor-adjusting means are provided (for example a potentiometer for setting the scale factor $P_C$ for determining a n-hexane gas-concentration reduced value $C_N$ of propane gas used in the sensitivity-calibration), the initial adjustment of the HC analyzer can be readily achieved by setting said scale factor $P_C$ at an optional specified numerical value, such as 0.500 (= 1/2) which can be easily determined by operating said scale factor-adjusting means. The further calculation of said n-hexane gas-concentration reduced value $C_N$ = (concentration $C_P$ of standard propane gas) x (scale factor $P_C$) can be achieved easily and with remarkably less figures in comparison with the conventional sensitivity-calibration circuit, for example 1,862 x 0.5 = 0.931.

In addition, if the present invention is applied to HC analyzers of the same kind, a scale factor $P_C$ of an optional specified numerical value (for example 0.500), which can be determined in the same manner as the above described, can be easily set in common for all of those HC analyzers. In such a case, the calibration can be carried out fast and simple without requiring the calculation at all by recording not only the concentration $C_p$ but also the product of the concentration $C_P$ of the standard propane and the common scale factor $P_C$ for all of said HC analyzers. In determining a n-hexane gas-concentration reduced value $C_N$ of the standard propane gas itself, only the information on a display label of a cylinder of standard propane gas used in the sensitivity-calibration after the initial adjustment of the HC analyzer is used.

Preferred embodiments of a sensitivity-calibration circuit in a HC analyzer according to the present invention are shown in the accompanying drawings, in which

FIG. 1 is a block diagram showing a circuit of one preferred embodiment; and

FIG. 2 is a block diagram showing a circuit in another preferred embodiment of the invention.

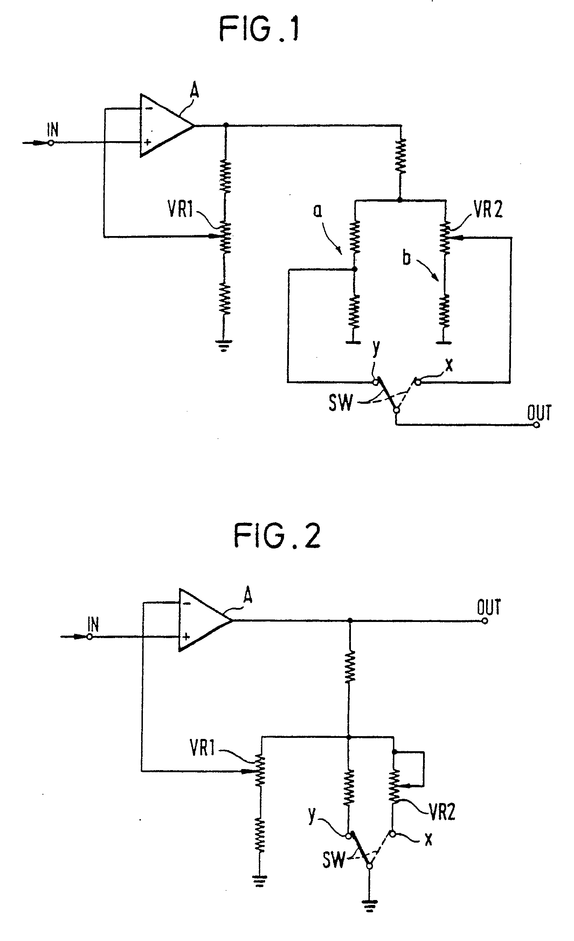

In FIG. 1 A designates an amplifier for amplifying a concentration-detecting signal (voltage) supplied from a HC analyzer (not shown) through an input terminal IN, VR1 designates a sensitivity-calibration potentiometer for adjusting the sensitivity by controlling the amplification factor of said amplifier A, and SW designates a switch which can be switched to a position y in which an output signal is supplied from said amplifier A to an output terminal OUT through a line a for a second side (shown by a full line), and a position x, in which the output signal is led from said amplifier A to the output terminal OUT through a line b of a first side (shown by a dotted line).

Said line b of the first side is provided with a trimmer VR2 composing scale factor-adjusting means VR2 capable of setting a scale factor $P_C$ in said analyzer for determining a n-hexane gas-concentration reduced value $C_N$ of propane gas used in the sensitivity-calibration of the HC analyzer at an optional value in the middle thereof.

The sensitivity-calibration of the HC analyzer constructed in the above described manner is carried out as follows:

At first, during the initial adjustment of the HC analyzer, an appointed sensitivity-calibration is carried out by switching over said switch SW to the second side (position y), flowing standard n-hexane gas having a known concentration through the HC analyzer, and adjusting said sensitivity-calibration potentiometer VR1 under such a condition so that an output signal corresponding to a known concentration can be obtained from said output terminal OUT.

Subsequently, said switch SW is switched over to the first side (position x) and standard propane gas also having a known concentration $C_P$ (for example 1,862 ppm) is passed through the HC analyzer. If the scale factor $P_C$ for determining the n-hexane gas-concentration reduced value $C_N$ of propane gas for the HC analyzer including the sensitivity-calibration circuit as a whole is set at an optional specified numerical value (for example 0.500 = 1/2) which can be easily calculated, the trimmer VR2 as said scale factor-adjusting means VR2 is adjusted so that an output signal may be obtained from said output terminal OUT corresponding to the n-hexane gas-concentration reduced value $C_N$ (= 931 ppm) determined on the basis of the above described equation:

n-hexane gas-concentration reduced value $C_N$
= (concentration $C_P$ of standard propane gas)
x (scale factor $P_C$);

e.g. $C_N$ = 1.862 x 0.5 = 0.931 (ppm), and then the trimmer VR2 is fixed.

Thus, since said scale factor $P_C$ in the HC analyzer is set at an optional specified numerical value (0.500 = 1/2) by which said calculation can be easily carried out, in the subsequent usual calibration the calculation on the basis of the above described equation:

N-hexane gas-concentration reduced value $C_N$
= (concentration $C_P$ of standard propane gas)
x (scale factor $P_C$)

can be easily carried out with remarkably less figures by the use of an optional specified numerical value (0.500 = 1/2) making the calculation easy.

In another preferred embodiment, as shown in FIG. 2, the amplification factor of the amplifier A is varied by switching over the switch SW between the second side y and the first side x.

With a sensitivity-calibration circuit in a HC analyzer having the constructional features shown in FIG. 2, a fundamentally similar effect to that of the above described circuit can be achieved with the low impedance output.

With a sensitivity-calibration circuit according to the present invention, due to the factor-adjusting means, a scale factor $P_C$ determining a n-hexane gas-concentration reduced value $C_N$ of propane gas used in the sensitivity-calibration of the analyzer can be set at an optional easily calculated numerical value, such as 0.500 (= 1/2), whereby in the usual calibration after the initial adjustment the calculation can be achieved with remarkably less figures in comparison with the conventional sensitivity-calibration circuit. In addition, if the present invention is applied to all of HC analyzers of the same kind, a scale factor $P_C$ of an optional specified numerical value (for example 0.500 ), can be easily set in common for all of those HC analyzers. In such a case, the calibration becomes remarkably simple and can be carried out quickly without requiring a calculation at all by recording not only the concentration $C_P$ but also a product of the concentration $C_P$ of the standard propane gas and the common scale factor $P_C$ for all of said HC analyzers. In short, a n-hexane gas-concentration reduced value $C_N$ of a standard propane gas itself, shown on a display label of a cylinder of standard propane gas,is all that is needed for the sensitivity-calibration after the initial adjustment of the HC analyzer has been accomplished.

## Claims

1. A sensitivity-calibration circuit for a hydrocarbon (HC) analyzer used for detecting the concentration of HC gases as an n-hexane reduced gas concentration $C_N$ of propane gas, said circuit being connected to an output of said analyzer and comprising a sensitivity adjusting means (VR1) for adjusting the sensitivity of said analyzer, **characterized in that** said circuit further comprises a scale factor adjusting means (VR2) for setting a scale factor $P_C$ of said analyzer and for determining said n-hexane reduced gas concentration value $C_N$ of propane gas, said scale factor adjusting means (VR2) being coupled to said sensitivity adjusting means (VR1) and to a switching means (SW) for switching said circuit between a first branch (b) including said scale factor adjusting means (VR2) and a second branch (a).

2. The sensitivity-calibration circuit as set forth in Claim 1, **characterized in that** said scale factor adjusting means (VR2) is provided at an output side of an amplifier (A) whose amplification factor is adjusted by said sensitivity adjusting means (VR1).

3. The sensitivity-calibration circuit as set forth in Claims 1 or 2, **characterized in that** said sensitivity adjusting means (VR1) is a potentiometer.

4. The sensitivity-calibration circuit as set forth in one of the Claims 1 to 3, **characterized in that** the scale factor adjusting means (VR2) is a trimmer.

5. The sensitivity calibration circuit as set forth in Claim 2, **characterized in that** the first branch (b) and the second branch (a) are connected to the output side of said amplifier (A).

6. The sensitivity-calibration circuit as set forth in Claim 2, **characterized in that** the amplification factor of said amplifier (A) is varied by switching over the circuit between the first branch (b) and the second branch (a).

## Revendications

1. Circuit d'étalonnage de sensibilité pour un analyseur d'hydrocarbures (HC) utilisé pour détecter la concentration d'hydrocarbures gazeux en tant que concentration de gaz $C_N$ ramenée à n-hexane de gaz propane, ledit circuit étant relié à une sortie dudit analyseur et comportant un moyen de réglage de sensibilité (VR1) pour régler la sensibilité dudit analyseur, caractérisé en ce que ledit circuit comporte en outre un moyen de réglage (VR2) de facteur de proportionnalité afin de régler un facteur de proportionnalité $P_C$ dudit analyseur et pour déterminer ladite valeur $C_N$ de concentration de gaz ramenée à n-hexane de gaz propane, ledit moyen de réglage de facteur de proportionnalité (VR2) étant relié audit moyen de réglage de sensibilité (VR1) et à un organe de commutation (SW) pour commuter ledit circuit entre une première dérivation (b) comprenant ledit moyen de réglage de facteur de proportionnalité (VR2) et une seconde dérivation (a).

2. Circuit d'étalonnage de sensibilité selon la revendication 1, caractérisé en ce que ledit moyen de réglage de facteur de proportionnalité (VR2) est disposé à la sortie d'un amplificateur (A) dont le gain est réglé par ledit moyen de réglage de sensibilité (VR1).

3. Circuit d'étalonnage de sensibilité selon les revendications 1 ou 2, caractérisé en ce que ledit moyen de réglage de sensibilité (VR1) est un potentiomètre.

4. Circuit d'étalonnage de sensibilité selon l'une quelconque des revendications 1 à 3, caractérisé en ce que le moyen de réglage de facteur de proportionnalité (VR2) est un rhéostat d'équilibrage.

5. Circuit d'étalonnage de sensibilité selon la revendication 2, caractérisé en ce que la première dérivation (b) et la seconde dérivation (a) sont reliées à la sortie dudit amplificateur (A).

6. Circuit d'étalonnage de sensibilité selon la revendication 2, caractérisé en ce que le gain dudit amplificateur (A) est modifié en commutant le circuit entre la première dérivation (b) et la seconde dérivation (a).

**Ansprüche**

1. Empfindlichkeits-Kalibrierschaltung für einen Kohlenwasserstoff (HC)-Analysator zum Detektieren der Konzentration eines HC Gases als n-Hexan reduzierte Gaskonzentration $C_N$ von Propangas, wobei die Schaltung mit einem Ausgang des Analysators verbunden ist und eine Empfindlichkeits-Einstelleinrichtung (VR1) zum Einstellen der Empfindlichkeit des Analysators aufweist, **dadurch gekennzeichnet,** daß die Schaltung ferner eine Skalenfaktor-Einstelleinrichtung (VR2) zum Einstellen eines Skalenfaktors $P_C$ des Analysators sowie zur Bestimmung des n-Hexan reduzierten Gaskonzentrationswertes $C_N$ von Propangas aufweist, wobei die Skalenfaktor-Einstelleinrichtung (VR2) mit der Empfindlichkeits-Einstelleinrichtung (VR1) sowie mit einem Umschalter (SW) gekoppelt ist, um in der Schaltung zwischen einem ersten Zweig (b), der die Skalenfaktor-Einstelleinrichtung (VR2) enthält, und einem zweiten Zweig (a) umschalten zu können.

2. Empfindlichkeits-Kalibrierschaltung nach Anspruch 1, **dadurch gekennzeichnet,** daß die Skalenfaktor-Einstelleinrichtung (VR2) an der Ausgangsseite eines Verstärkers (A) vorhanden ist, dessen Verstärkungsfaktor durch die Empfindlichkeits-Einstelleinrichtung (VR1) einstellbar ist.

3. Empfindlichkeits-Kalibrierschaltung nach Anspruch 1 oder 2, **dadurch gekennzeichnet,** daß die Empfindlichkeits-Einstelleinrichtung (VR1) ein Potentiometer ist.

4. Empfindlichkeits-Kalibrierschaltung nach einem der Ansprüche 1 bis 3, **dadurch gekennzeichnet,** daß die Skalenfaktor-Einstelleinrichtung (VR2) ein Trimmer ist.

5. Empfindlichkeits-Kalibrierschaltung nach Anspruch 2, **dadurch gekennzeichnet,** daß der erste Zweig (b) und der zweite Zweig (a) mit der Ausgangsseite des Verstärkers (A) verbunden ist.

6. Empfindlichkeits-Kalibrierschaltung nach Anspruch 2, **dadurch gekennzeichnet,** daß der Verstärkungsfaktor des Verstärkers (A) durch Umschalten der Schaltung auf den ersten Zweig (b) oder den zweiten Zweig (a) veränderbar ist.

# FIG.1

# FIG.2